# EUROPEAN PATENT APPLICATION

(11) **EP 4 613 188 A1**
(43) Date of publication of application: **10.09.2025**
(21) Application number: 24162077.2
(22) Date of filing: 07.03.2024
(51) Int. Cl.: A61B 5/024, A61B 5/055, A61B 5/103, A61B 5/107, A61B 5/11, A61B 5/113, G01L 1/24

(54) **MONITORING PATIENT STATE DURING MEDICAL IMAGING**

(71) Applicant: Koninklijke Philips N.V., 5656 AG Eindhoven (NL); Luxisens Technologies B.V., 5221 EC Den Bosch (NL)
(72) Inventor: DENISSEN, Adrianus Johannes Maria, Eindhoven (NL); VAN DEN BOOM, Henricus Petrus Anna, Eindhoven (NL); HEUVELINK, Annerieke, 5656AG Eindhoven (NL)
(74) Representative: Philips Intellectual Property & Standards

(57) **Abstract**

There is provided a method for monitoring patient state during medical imaging. The method comprises: obtaining pressure signals from at least one optical waveguide pressure sensor array arranged to interact with the patient during an image acquisition process; and processing the pressure signals to monitor patient state. A corresponding patient state monitoring system is also provided.

## Description

### FIELD OF THE INVENTION

The invention relates to methods and systems for monitoring patient state during medical imaging consisting of an MR, CT, Xray procedure or on an RT-table.

### BACKGROUND OF THE INVENTION

Pressure sensing systems which rely on the use of optical fibers as the sensor, such as that described in WO 2017/144675 A1, have been used for monitoring movements of persons during sleep. However, the full potential of such systems is yet to be realized.

### SUMMARY OF THE INVENTION

To better address one or more of these concerns, there is provided, in a first aspect of the invention, a method as defined by the following non-exhaustive list of non-limiting examples. Any one or more of the features of these examples may be combined with any one or more features of any other example, embodiment, or aspect described herein.

A method for monitoring patient state during medical imaging, the method comprising:
obtaining pressure signals from at least one optical waveguide pressure sensor array arranged to interact with the patient during an image acquisition process; and
processing the pressure signals to monitor patient state.

In particular, processing the pressure signals may comprise detecting patient motion. Detecting patient motion may comprise detecting motion of one or more body parts of the patient. Detecting motion of one or more body parts of the patient may comprise detecting motion of one or more of a wrist, forearm, humerus, elbow, collarbone, foot, ankle, tibia-fibula, femur, chest, lung, rib, hip, pelvis, abdomen, spine, skull, upper airway, jaw, mandible, sinus, head, neck, knee, shoulder, leg, and/or a hand of the patient.

The medical imaging may comprise diagnostic X-ray imaging - DXR.

Detecting patient motion may comprise detecting patient head movement. Detecting patient head movement may comprise detecting patient head rotation.

Detecting patient motion may comprise estimating a magnitude of the detected patient motion. Detecting patient motion may comprise estimating an origin of the detected patient motion.

The method may further comprise performing a calibration procedure to determine a relationship between one or more predetermined patient motions or positions and the obtained pressure signals. The one or more predetermined patient motions may comprise one or more head movements.

Obtaining the pressure signals from the at least one optical waveguide pressure sensor array may comprise obtaining pressure signals from at least first and second said optical waveguide pressure sensor arrays arranged to interact with the patient. The method may further comprise overlaying the first optical waveguide pressure sensor array on the second optical waveguide pressure sensor array before obtaining the pressure signals therefrom. The overlaying may comprise arranging the first optical waveguide pressure sensor array so that its receiving optical waveguides are oriented orthogonally to receiving optical waveguides of the second optical waveguide pressure sensor array. The overlaying may comprise arranging one or both of the first and second optical waveguide pressure sensor arrays so that its receiving fibers are aligned with one or more expected directions of patient motion.

Detecting patient motion may comprise detecting patient respiratory activity. Detecting patient motion may comprise detecting patient cardiac activity. Detecting patient motion may comprise detecting patient cardiorespiratory activity. Detecting the patient motion may comprise detecting patient respiratory activity and patient cardiac activity simultaneously.

Processing the pressure signals to monitor patient state may comprise determining one or more patient body measurements. The body measurements may comprise patient body weight. The body measurements may comprise one or more geometric measurements. The body measurements may comprise patient body height. The method may further comprise detecting a change in one or more of the body measurements. Detecting the change may comprise detecting a change in patient body weight. Detecting the change may comprise detecting a change in patient body height. Detecting the change may comprise detecting an interfractional change.

Processing the pressure signals to monitor patient state may comprise locating one or more patient organs. Processing the pressure signals to monitor patient state may comprise determining a level of filling of one or more patient organs. The one or more patient organs may comprise a bladder. The one or more patient organs may comprise a rectum. Detecting the change in the one or more body measurements may comprise detecting a change in the level of filling of the one or more patient organs.

Processing the pressure signals to monitor patient state may comprise comparing a first patient position determined at a first timepoint with a second patient position determined at a second timepoint to identify a deviation in patient position. The first patient position may comprise a previous patient position used during a previous image acquisition process. The first patient position may serve as a reference patient position for subsequent monitoring. The second patient position may comprise a current patient position. Detecting patient respiratory activity may comprise detecting patient respiratory activity at multiple locations. The multiple locations may comprise locations in the chest and/or abdomen of the patient. The method may further comprise using the detected respiratory activity to estimate motion of one or more patient organs. Detecting patient respiratory activity comprises using calibration data in the detection. The method may further comprise using the detected patient respiratory activity to characterize patient breathing. Detecting patient motion may comprise detecting patient bowel motion. Detecting patient motion may comprise detecting patient motion in two or more dimensions. The method may further comprise using the detected patient motion in two or more dimensions to detect changes in patient respiration. The method may further comprise using the detected patient motion in two or more dimensions to detect changes in patient respiration from chest-to-belly breathing to back breathing or vice versa.

The method of the first aspect may be at least partially computer implemented.

According to a second aspect, there is provided a computing system configured to perform the method of the first second aspect. The computing system may comprise, or be comprised in, the patient state monitoring system described herein.

According to a third aspect, there is provided a computer program comprising instructions which, when executed by a computing system, enable or cause the computing system to perform the method of the first aspect.

According to a fourth aspect, there is provided a computer-readable medium comprising instructions which, when executed by a computing system, enable or cause the computing system to perform the method of the first aspect. The computer-readable medium may be transitory or nontransitory, volatile or non-volatile.

By monitoring patient state during medical imaging using at least one optical waveguide pressure sensor array arranged to interact with the patient during an image acquisition process, in the manner described herein, image quality can be improved and the efficiency of radiotherapy of moving organs increased.

The pressure sensing system as described herein can measure ballistic heartbeat and small weight changes during breathing in multiple dimensions.

By detecting breathing and heart rate using the pressure sensing system as described herein, no sensors need be attached to the patient, which provides less montage work for the technologist and more comfort for the patient. Moreover, the body shape contour is no longer disturbed by the mounting of a respiration belt and image quality is no longer disturbed by ECG stickers or wires. Additionally, no extra scan sequences are needed to estimate the position on the diaphragm (the so-called navigator).

As used herein, the term "patient" refers to the human or animal subject of a medical imaging examination and does not necessarily denote a person of ill health. That is, the term "patient" may be applied to the subject regardless of the state of health of the subject or the environment in which the medical imaging is performed.

As used herein, the term "patient state" may comprise one or more of: a position of the patient or part thereof; a motion of the patient or part thereof; a condition of the patient or part thereof. The patient state may comprise for example a medical state of the patient, as indicated for example by one or more vital signs, or a state or condition of one or more biological processes of the patient, for example as indicated by one or more biomarkers. In particular examples described herein, "patient state" may be used to refer to the sedation level of the patient, the state of the cardiorespiratory activity of the patient, the state of internal organs of the patient, for example the filling level of one or more internal organs, e.g. bowel, bladder, and so on. As an example of patient condition, the patient state may comprise a patient geometry, that is, one or more body measurements or geometrical measurements relating to the patient. Such measurements may relate to one or more physiological parameters and/or anthropometric parameters characterizing the patient, e.g. dimensional descriptors of body size and shape, e.g. height, weight, body mass index (or BMI), waist-to-hip ratio, and so on.

As used herein, the term "patient position", as one example of patient state, may refer in particular to a location and/or posture of the patient or part thereof. The location and/or posture may be defined relative to one or more references, for example a previous location or posture, some part of the imaging apparatus, such as the patient support, or another part of the patient. The patient position may alternatively be referred to herein as a "pose".

Accordingly, the term "patient motion" may be understood in terms of a deviation in patient position between two or more different timepoints. "Detecting patient motion" as described herein may thus comprise obtaining pressure signals corresponding to two or more different timepoints, and detecting patient motion based on one or more differences between the pressure signals corresponding respectively to the two or more different timepoints. To that end, the pressure signals may therefore be obtained continuously, periodically, or repetitively. In this way, detecting patient motion may comprise tracking patient position. The obtained pressure signals may thus comprise both spatial and temporal data, i.e. spatiotemporal data. The pressure signals may thus be used to obtain a static or dynamic occupancy pressure distribution whereby patient state including patient position and/or patient motion may be detected. Patient motion as described herein may be interfractional, that is, variations in patient position occurring from one treatment fraction to the next, possibly because of variability in patient positioning or volumetric changes in a tumor, for example. Additionally or alternatively patient motion may by interfractional, that is, variations in patient position occurring during a single treatment fraction, causing geometric alterations from moment to moment. Patient motion may comprise intentional and/or unintentional patient movement, that is, conscious and/or unconscious patient motion. Intentional or conscious patient motion may comprise deliberate position changes or user gestures for communication purposes, for example. Unintentional or unconscious patient motion may comprise for example internal motion, usually organ motion, resulting from physiological processes such as cardiorespiratory activity (respiration, heartbeat), organ filling, gastrointestinal activity such as peristalsis, or urinary activity. Organ motion may be defined as the mobility of organs relative to the bony anatomy, for example. Patient motion may thus involve micromovements and/or macromovements.

In contrast to "patient position", the term "patient positioning" as used herein refers to the act of putting the patient into a desired patient position on the patient support. Patient positioning may alternatively be referred to herein as "posing".

By "patient support" is meant that part of the imaging apparatus which supports the patient, for example an examination table.

As used herein, the term "monitoring" refers to the acquisition of data defining the patient state at at least one point in time, preferably at several timepoints, for example in a continuous, periodic or repetitive manner.

As used herein, the term "medical imaging" refers to the process of imaging at least part of the interior or exterior of a body for clinical analysis and medical intervention, and comprises techniques such as radiography, magnetic resonance imaging, ultrasound, computed tomography, and so on.

As used herein, the term "quantitative imaging" refers to the extraction of quantifiable features from medical images for the assessment of normal or of the severity, degree of change, or status of a disease, injury, or chronic condition relative to normal.

As used herein, the term "remedial action" refers to one or more corrective actions and/or preventive actions and/or actions to discard unwanted information. For example, taking remedial action may comprise stopping, suspending, updating, or repeating at least part of an imaging plan or treatment plan, and/or interacting with one or more users, for example by issuing a warning, providing a report, or providing user output, and so on.

The term "circuitry", as used herein, may comprise, for example, singly or in any combination, hardwired circuitry, programmable circuitry such as computer processors comprising one or more individual instruction processing cores, state machine circuitry, and/or firmware that stores instructions executed by programmable circuitry.

The term "obtaining", as used herein, may comprise, for example, receiving from another system, device, or process; receiving via an interaction with a user; loading or retrieving from storage or memory; measuring or capturing using sensors or other data acquisition devices.

The term "determining", as used herein, encompasses a wide variety of actions, and may comprise, for example, calculating, computing, processing, deriving, investigating, looking up (e.g., looking up in a table, a database or another data structure), ascertaining, and the like. Also, "determining" may comprise receiving (e.g., receiving information), accessing (e.g., accessing data in a memory), and the like. Also, "determining" may comprise resolving, selecting, choosing, establishing and the like.

The indefinite article "a" or "an" does not exclude a plurality. In addition, the articles "a" and "an" as used herein should generally be construed to mean "one or more" unless specified otherwise or clear from the context to be directed to a singular form.

Unless specified otherwise, or clear from the context, the phrases "one or more of A, B and C", "at least one of A, B, and C", and "A, B and/or C" as used herein are intended to mean all possible permutations of one or more of the listed items. That is, the phrase "A and/or B" means (A), (B), or (A and B), while the phrase "A, B, and/or C" means (A), (B), (C), (A and B), (A and C), (B and C), or (A, B, and C).

The term "comprising" does not exclude other elements or steps. Furthermore, the terms "comprising", "including", "having" and the like may be used interchangeably herein.

The invention may include one or more aspects, examples or features in isolation or combination whether specifically disclosed in that combination or in isolation. Any optional feature or sub-aspect of one of the above aspects applies as appropriate to any of the other aspects.

These and other aspects of the invention will be apparent from and elucidated with reference to the embodiments described hereinafter.

### BRIEF DESCRIPTION OF THE DRAWINGS

A detailed description will now be given, by way of example only, with reference to the accompanying drawings, in which:
Fig. 1 is a schematic diagram of a pressure sensing system;
Figs. 2A-Z illustrate pressure sensing systems such as that shown in Fig. 1 being used to sense pressure exerted by various anatomical regions of a patient in various different positions;

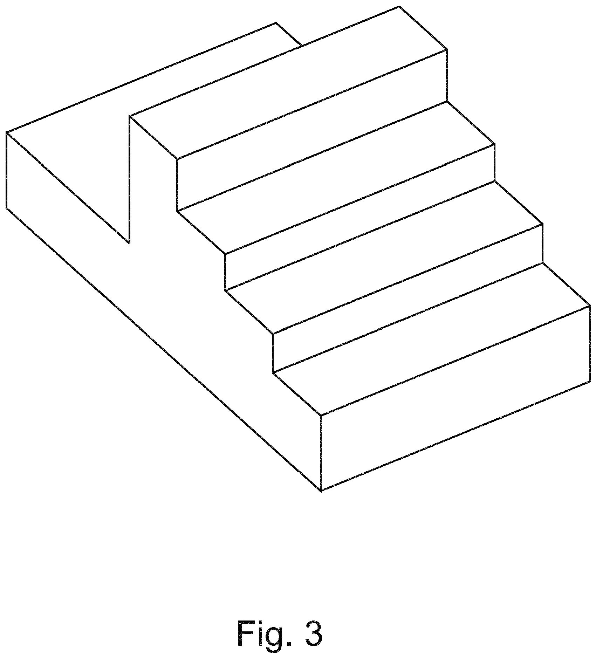

Fig. 3 illustrates pose assistance material which may be used to facilitate detection of patient motion using the pressure sensing system of Fig. 1;
Fig. 4 illustrates patient head rotation;
Fig. 5A and Fig. 5B illustrate the structure of an optical waveguide pressure sensor array;
Fig. 6 illustrates cardiorespiratory activity detected by the pressure sensing system of Fig. 1;

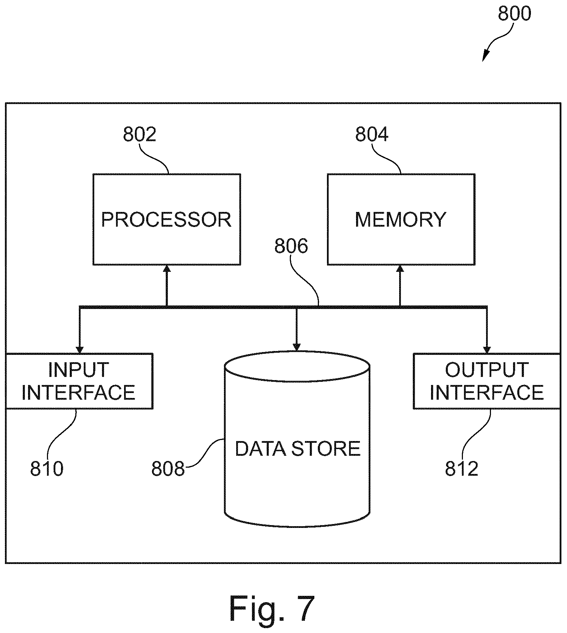

Fig. 7 illustrates a computing system that can be used in accordance with the systems and methods disclosed herein.

### DETAILED DESCRIPTION OF EMBODIMENTS

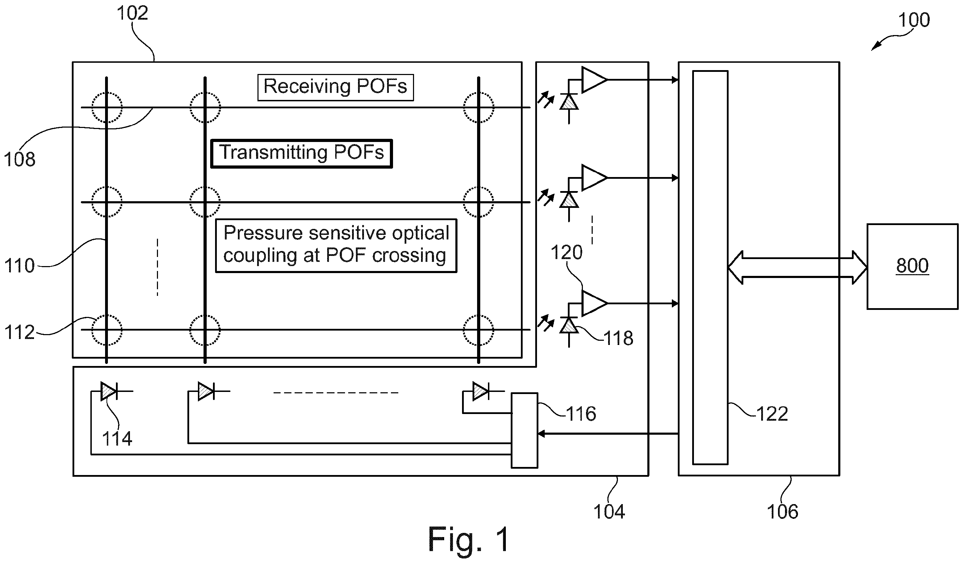

Fig. 1 is a schematic diagram of a pressure sensing system 100. The pressure sensing system 100 comprises an optical waveguide pressure sensor array 102, optoelectronic circuitry 104, and data acquisition and control circuitry 106. In some contexts, the pressure sensing system 100 may be referred to as a "sensemat".

The optical waveguide pressure sensor array 102 comprises a plurality of row optical waveguides, such as that indicated at 108, and a plurality of column optical waveguides, such as that indicated at 110. The optical waveguides 108, 110 preferably comprise polymer or plastic optical fibers (POF). The row optical waveguides 108 and the column optical waveguides 110 are deformable and are arranged in a planar array or matrix to define a sensor at each crosspoint, such as that indicated at 112. Each crosspoint 112 thus includes one of the row optical waveguides 108 in contact with one of the column optical waveguides 110 at the intersection of those two waveguides. Each crosspoint 112 may further comprise a light coupling structure (not shown) configured to enhance waveguide bending when pressure is applied to the crosspoint 112. The light coupling structure may comprise at least one layer of mechanical light scattering material disposed in contact with at least one of the row or column optical waveguides 108, 110. The light coupling structures may be applied on both sides of the row and column optical waveguides 108, 110, and may have a layer of light scattering material either on one side, or on both sides. In one non-limiting example, each row optical waveguide 108 includes a waveguide core surrounded by a waveguide cladding, and each column optical waveguide 110 includes a waveguide core surrounded by a waveguide cladding, wherein the waveguide claddings of both the row optical waveguides 108 and the column optical waveguides 110 are arranged for light transmissive contact at one or more of the crosspoints 112. The cladding is sufficiently thin that a non-negligible amount of light may be coupled from the core of one waveguide to the crossing waveguide. At an increased ratio between cross-sectional diameter of the core to the cladding, the light has a higher tendency to couple into the crossing waveguide. As such, the ratio may be at least 50:1 but more preferably is higher.

The optoelectronic circuitry 104 comprises a plurality of LEDs 114, with each LED 114 being connected to a respective one of the column optical waveguides 110, which thereby serve as transmitting waveguides. A selector 116 is configured to enable selective activation of the LEDs 114 by the data acquisition and control circuitry 106. The optoelectronic circuitry 104 further comprises a plurality of photodiodes 118, each coupled to a respective row optical waveguide 108 to receive light therefrom. The row optical waveguides 108 thus serve as receiving optical waveguides. It will, however, be understood that the column optical waveguides 110 may alternatively serve as the receiving optical waveguides and that the row optical waveguides 108 may serve as the transmitting optical waveguides. The optoelectronic circuitry 104 further comprises a plurality of amplifiers 120 for amplifying the signals produced by respective photodiodes 118. Since the optical coupling between transmitting and receiving optical waveguides is small, high-sensitivity optical receivers are needed. The amplifiers 120 may therefore comprise transimpedance amplifiers (TIAs) with high gain and a high input impedance. The amplified signals are output to the data acquisition and control circuitry 106.

The data acquisition and control circuitry 106 controls the LEDs 114 and processes the signals obtained from the photodiodes 118. To that end, the data acquisition and control circuitry 106 comprises interface circuitry 122 for interfacing with the optoelectronic circuitry 104. The interface circuitry 122 may comprise one or more analog-to-digital converts for converting the analog signals output by the amplifiers 120 to digital signals. Preferably, for improving the SNR of sensors, the LEDs are designed and/or controlled so that the full range of the analog-to-digital converters can be used. The digital pressure signals may then be output to a patient state monitoring system 800 for further processing as described hereinbelow. The patient state monitoring system 800 and/or the data acquisition and control circuitry 106 may be configured to perform one or more preprocessing operations, such as resizing, orienting, scaling, filtering, segmenting, reducing noise, and so on, for rendering the pressure signals more suitable for the further processing operations.

In use, the optical waveguide pressure sensor array 102 is configured to sense pressure, when light is provided to the row optical waveguides 108, by measuring the light coupled at each crosspoint 112 to its column optical waveguide 110 (or vice versa). The amount of coupled light is dependent on the pressure applied to the crosspoint 112, which thereby acts as a pressure sensor. Moreover, as only a small amount of light is coupled out of the transmitting optical waveguide 110 when pressure is exerted on the respective crosspoint 112, the light power which remains in the transmitting optical waveguide 110 and which arrives at the next crosspoint 112 is hardly reduced. Thus, the sensitivity of a crosspoint 112 is hardly affected by the pressure on other crosspoints 112, i.e. the location-dependence of the performance of a crosspoint 112 is negligible. There is therefore provided a two-dimensional optical waveguide pressure sensor array 102 having high sensitivity. With such a pressure sensor array 102, the two-dimensional spatial distribution of pressure of objects and/or persons on a certain structure can be measured.

In one example of a pressure sensing operation performed by the pressure sensing system 100, a crosspoint scanning method is implemented. The data acquisition and control module 106 controls the selector 116 to activate only one LED 114 at a time and scans the crosspoints 112 column-by-column. Reading the amplified signals output by the photodiodes 118 line-by-line enables the data acquisition and control circuitry 106 to perform 2D pressure sensing. This solution is readily scalable because N photodiodes and M LEDs can sense pressure at N x M crosspoints when the columns are scanned one-by-one.

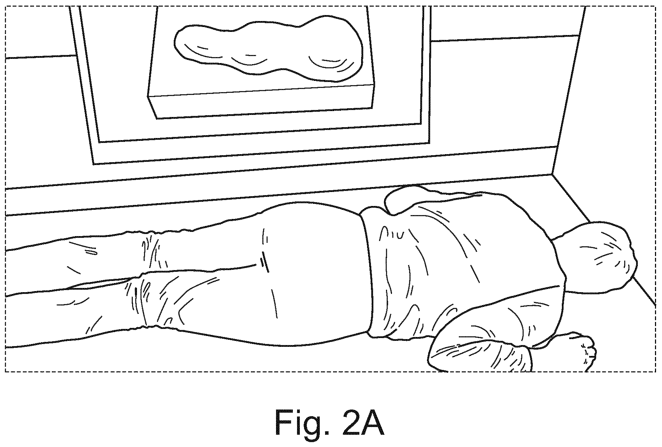

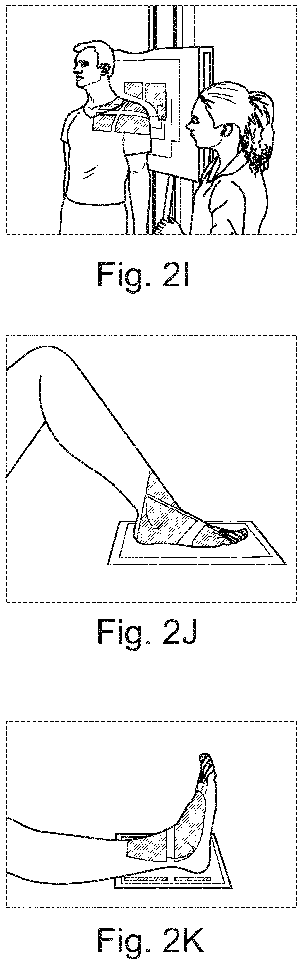

During the image acquisition process, the pressure sensor array 102 is arranged to interact with the patient such that the pressure signals may represent the patient state. That is, the sensor array 102 is positioned for direct or indirect contact with at least part of the patient, i.e., with at least one body part of the patient, preferably that part which is relevant for the subsequent processing as described below. By "indirect" is meant that there may be intervening objects between the patient and the sensor array 102. For example, Fig. 2A depicts the pressure sensed by the pressure sensing system 100 when positioned underneath a mattrass with the patient lying on the mattrass. The pressure sensing system 100 is not prevented by the indirect contact from outputting useful information by virtue of its sensitivity. In Fig. 2A, the patient state monitoring system 800 is shown as obtaining the pressure signals from the pressure sensing system 100 and displaying a representation of the 2D sensed pressure on a display. As described further below, the pressure sensing system 100 and/or the patient state monitoring system 800 may comprise one or more user interface components for interfacing with the user, such as a technologist and/or a patient.

According to the present disclosure, the pressure sensing system 100 is used for monitoring patient state during medical imaging to improve picture quality, speed and comfort. By installing the pressure sensing system 100 on or in the table of a CT, MR or Xray scanner for radiotherapy, the state of the patient (such as body position and respiration) can be monitored and also harmonized across different imaging modalities. Since the pressure sensing system 100 is thin (e.g. around 1mm thickness), flexible, and robust, it can be attached to the MR, CT, X-ray or RT table, for example by adhesive, without interfering with the imaging or radiation system. Aspects of the patient state such as the beating heart, respiration and body movement of the patient thereby become detectable. The present disclosure envisages numerous different use cases for the pressure sensing system 100 in the field of medical imaging, as will now be described.

### Use case #1: detecting patient motion

The patient can perform subtidal motion without noticing during image acquisition, which can lower the image quality. For example, movement of the legs during a head scan can disturb the homogeneity of the magnetic field and thereby lower the image quality. Even MR-generated peripheral nerve stimulation (PNS, see http://mriquestions.com/nerve-stimulation.html) could be detected, which is generally not observed by the technologist or noticeable by the patient. The pressure sensing system 100 can detect these movement phenomena.

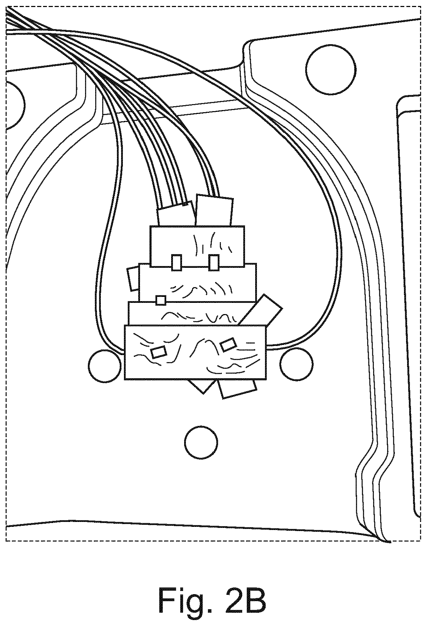

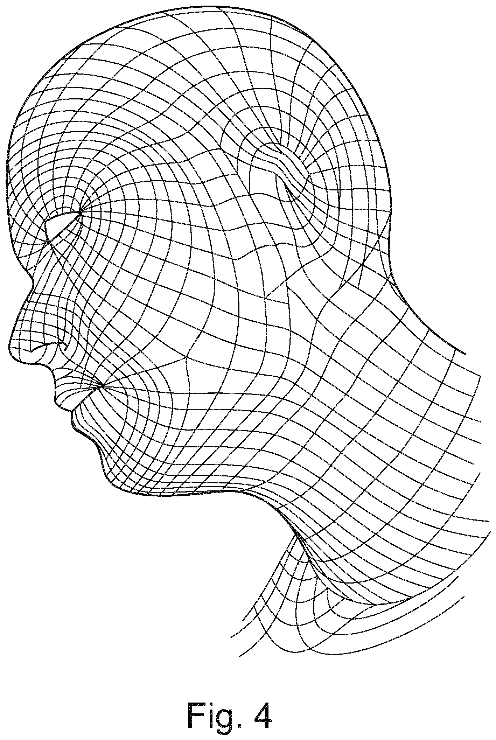

For scanning sequences that are relatively long for the patient, for example up to 45 minutes, it is very hard for the patient to lay perfectly still. Using a pressure sensing system such as that described with reference to Fig. 1 positioned underneath the pillow of the MR head coil, as depicted in Fig. 2B, it is possible to detect small head movements and head rotations, as illustrated in Fig. 4. In one non-limiting example, the pressure sensing system is able detect translations with an accuracy of 0.1mm and rotations with an accuracy of 0.1 degree. A 4D head calibration sequence during which the patient for instance slowly shakes their head to indicate "yes" and "no" for 30 seconds is enough to relate head movements to the signals output by the pressure sensing system 100.

The pressure sensing system 100 may be specially configured to monitor movement of the patient's head-and-neck, knee, shoulder, legs or hands, for example by its being sized and shaped to embed in the special coils. The pressure sensing system 100 is sufficiently sensitive to be able to detect swallowing, coughing, talking, singing and yawning, small movements which often result in bad image quality, especially for specialized sequences such as diffusion weighted imaging.

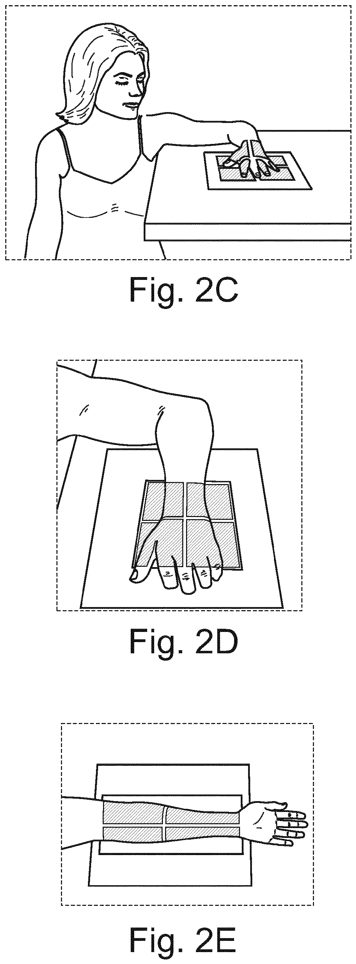

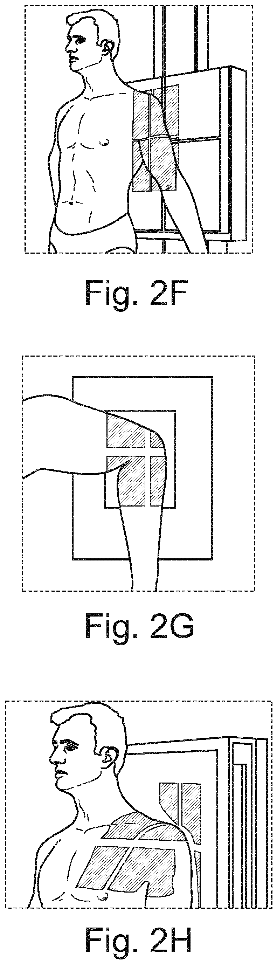

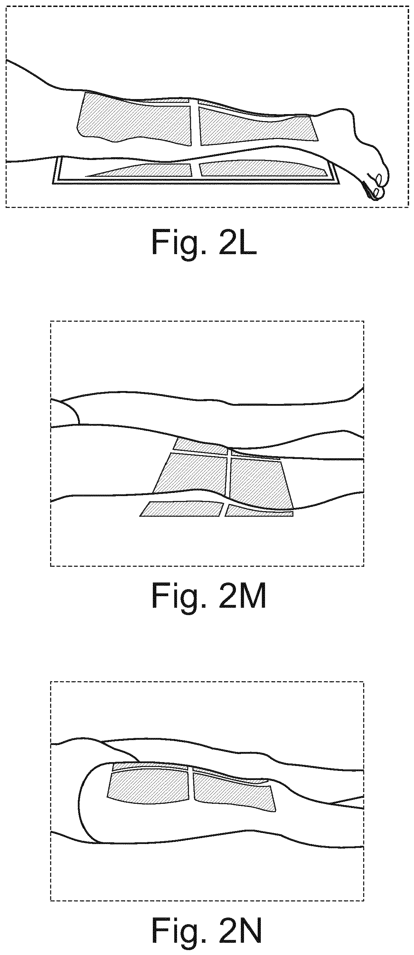

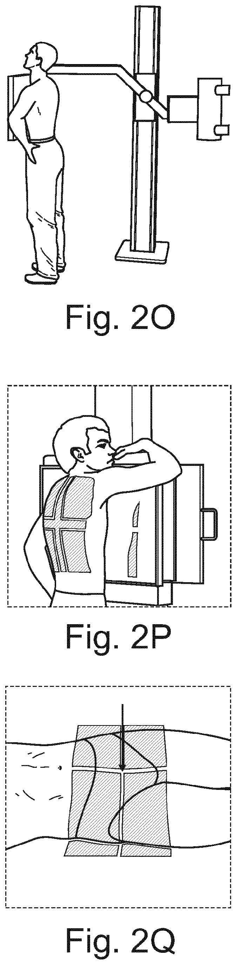

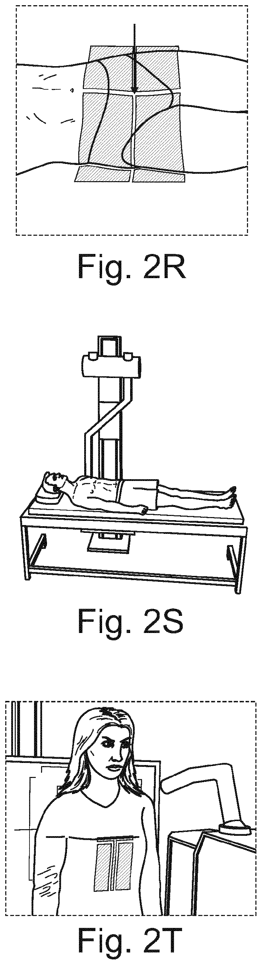

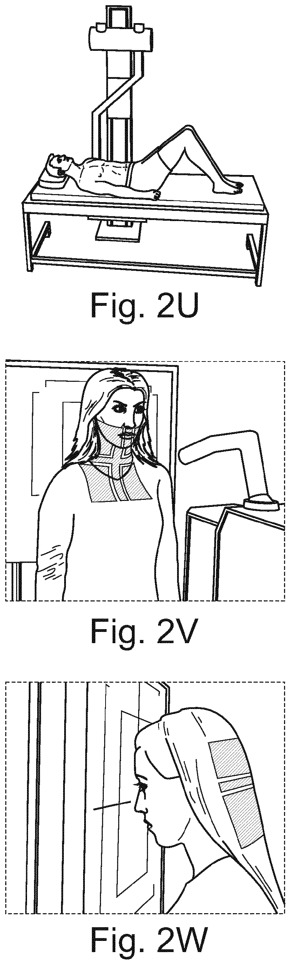

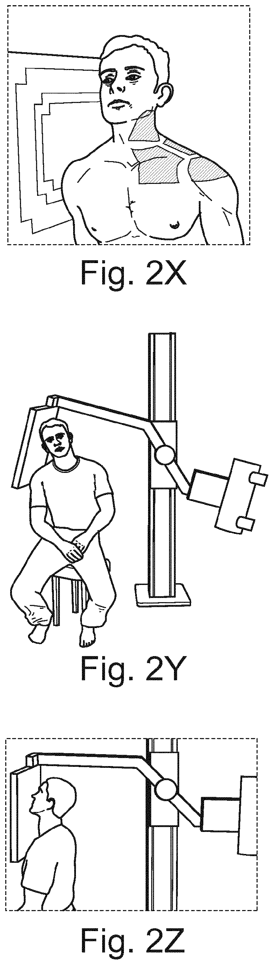

Figs. 2A-2Z illustrate the pressure sensing system 100 being used to sense pressure exerted by various anatomical regions of a patient in various different positions for the purposes of diagnostic X-ray imaging. For correct diagnostic medical imaging, e.g. diagnostic X-ray imaging (DXR), images of the part of the body that needs inspection should be positioned in the right way for optimal detection of problems. Suitable positions are illustrated in these figures for diagnostic medical imaging of the hand (Fig. 2C), wrist (Fig. 2D), forearm (Fig. 2E), humerus (Fig. 2F), elbow (Fig. 2G), shoulder (Fig. 2H), collarbone (Fig. 21), foot (Fig. 2J), ankle (Fig. 2K), tibia-fibula (Fig. 2L), knee (Fig. 2M), femur (Fig. 2N), chest (lung) (Fig. 2O), rib (Fig. 2P), hip (Fig. 2Q), pelvis (Fig. 2R), abdomen (Fig. 2S), spine (Figs. 2T, 2U), neck (Fig. 2V), skull (Fig. 2W), upper airway (Fig. 2X), jaw (mandible) (Fig. 2Y), and sinus (Fig. 2Z).

In nearly all poses (such as the DXR poses shown in Figs. 2C-2Z), the patient exerts some pressure which can be detected by the pressure sensing system 100. For example, the patient may exert pressure on the X-ray detector, which can be detected by the pressure sensing system 100 mounted on the detector.

### Use case #1a: detecting patient motion using layered pressure sensing systems

The thinness of the pressure sensing system 100 allows several dedicated pressure sensing systems to be deployed on top of each other and without interfering each other, as long as the active sensors do not overlap too much. Such a multi-layered pressure sensing system can be used to increase resolution or observations, for example to capture user input, as described below with reference to use case #7. In particular, the sensitivity of the pressure sensing system 100 can be improved by putting two (or more) layers of waveguides 108, 110 on top of each other. One possibility is to have the direction of the top-layer receiving optical waveguides 108 orthogonal to the direction of the bottom-layer receiving optical waveguides 108. Another possibility is to arrange the direction of both layers of receiving optical waveguides 108 in such a way that they are aligned with the most likely direction of movement of the body part involved. For instance, for detecting lateral rotation of the head in the MR scanner, the receiving optical waveguides in both or all layers may be substantially aligned with the direction of this motion.

### Use case #2: improvements to the pressure sensing system

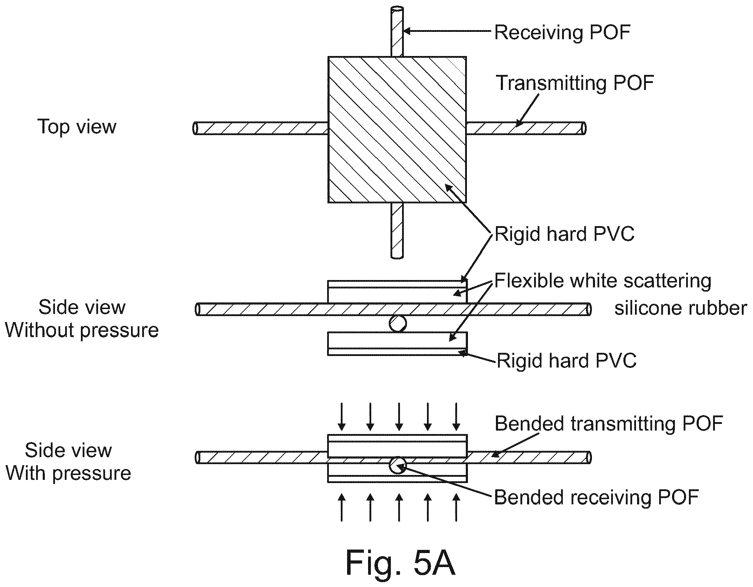

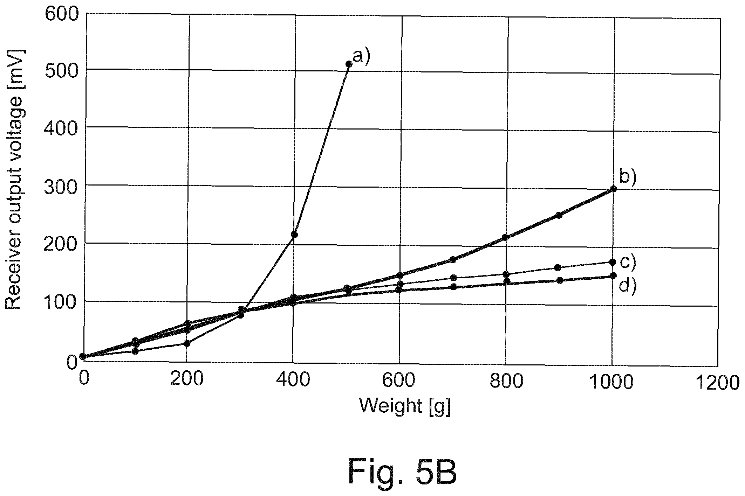

Replacement of the ring with free POF's can avoid hysteresis. A specific crossing construction can increase Signal to Noise Ratio (SNR). There can also be improvements to light source and detection circuit. This is illustrated in Figs. 5A and 5B.

### Use case #3: detection of breathing and beating heart

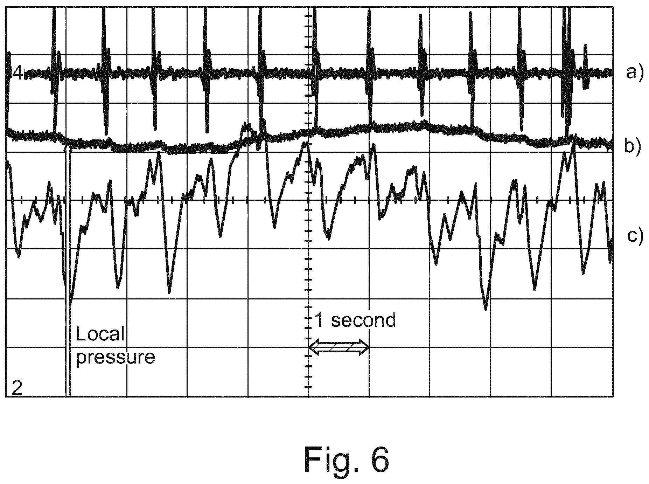

The pressure sensing system 100 may be located between the mattrass and the examination table to measure respiration and heart beating simultaneously, so as to produce signal output as shown in Fig. 6.

### Use case #4: detecting patient body weight and height

The patient state monitoring system 800 may be configured to estimate the body weight and height of the patient on the basis of the signals output by the pressure sensing system 100. The patient state monitoring system 800 may be configured to estimate the position of the organs. This saves time for the technologist to register the correct weight and height of the patient.

Moreover, the patient state monitoring system 800 may be configured to detect significant changes in weight or height, caused by for instance cancer treatment or by inflammation.

Using the signals output by the pressure sensing system 100, the patient state monitoring system 800 may be configured to estimate the filling of the bladder and/or rectum before and during imaging or RT.

### Use case #5: breathing characterization

The pressure sensing system 100 can detect respiration at multiple locations in the chest and/or abdomen of the patient.

Use case #6: monitor slow changing digestion processes like bladder or rectum fulling during imaging or RT

On the basis of signals output by the pressure sensing system 100, the patient state monitoring system 800 can estimate the filling of the bladder and/or rectum. In addition, the patient state monitoring system 800 can detect the motion of the bowel.

### Use case #7: motion characterization

The 2D design of the pressure sensing system 100 allows patient motion to be detected in more than one direction, which is generally done for so-called "motion surrogates".

Fig. 8 illustrates an exemplary computing system 800 that can be used in accordance with the systems and methods disclosed herein. In particular, the computing system may be used to implement the patient state monitoring system 800 described herein. The computing system 800 may form part of or comprise any desktop, laptop, server, or cloud-based computing system. The computing system 800 includes at least one processor 802 that executes instructions that are stored in a memory 804. The instructions may be, for instance, instructions for implementing functionality described as being carried out by one or more components described herein or instructions for implementing one or more of the methods described herein. The processor 802 may access the memory 804 by way of a system bus 806. In addition to storing executable instructions, the memory 804 may also store conversational inputs, scores assigned to the conversational inputs, etc.

The computing system 800 additionally includes a data store 808 that is accessible by the processor 802 by way of the system bus 806. The data store 808 may include executable instructions, log data, etc. The computing system 800 also includes an input interface 810 that allows external devices to communicate with the computing system 800. For instance, the input interface 810 may be used to receive instructions from an external computer device, from a user, etc. The computing system 800 also includes an output interface 812 that interfaces the computing system 800 with one or more external devices. For example, the computing system 800 may display text, images, etc. by way of the output interface 812.

It is contemplated that the external devices that communicate with the computing system 800 via the input interface 810 and the output interface 812 can be included in an environment that provides substantially any type of user interface with which a user can interact. Examples of user interface types include graphical user interfaces, natural user interfaces, and so forth. For instance, a graphical user interface may accept input from a user employing input device(s) such as a keyboard, mouse, remote control, or the like and provide output on an output device such as a display. Further, a natural user interface may enable a user to interact with the computing system 800 in a manner free from constraints imposed by input device such as keyboards, mice, remote controls, and the like. Rather, a natural user interface can rely on speech recognition, touch and stylus recognition, gesture recognition both on screen and adjacent to the screen, air gestures, head and eye tracking, voice and speech, vision, touch, gestures, machine intelligence, and so forth.

Additionally, while illustrated as a single system, it is to be understood that the computing system 800 may be a distributed system. Thus, for instance, several devices may be in communication by way of a network connection and may collectively perform tasks described as being performed by the computing system 800.

Various functions described herein can be implemented in hardware, software, or any combination thereof. If implemented in software, the functions can be stored on or transmitted over as one or more instructions or code on a computer-readable medium. Computer-readable media include computer-readable storage media. Computer-readable storage media can be any available storage media that can be accessed by a computer. By way of example, and not limitation, such computer-readable storage media can comprise FLASH storage media, RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Disk and disc, as used herein, include compact disc (CD), laser disc, optical disc, digital versatile disc (DVD), floppy disk, and Blu-ray disc (BD), where disks usually reproduce data magnetically and discs usually reproduce data optically with lasers. Further, a propagated signal may be included within the scope of computer-readable storage media. Computer-readable media also includes communication media including any medium that facilitates transfer of a computer program from one place to another. A connection, for instance, can be a communication medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio and microwave are included in the definition of communication medium. Combinations of the above should also be included within the scope of computer-readable media.

Alternatively, or in addition, the functionally described herein can be performed, at least in part, by one or more hardware logic components. For example, and without limitation, illustrative types of hardware logic components that can be used include Field-programmable Gate Arrays (FPGAs), Program-specific Integrated Circuits (ASICs), Program-specific Standard Products (ASSPs), System-on-a-chip systems (SOCs), Complex Programmable Logic Devices (CPLDs), etc.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features.

It has to be noted that embodiments of the invention are described with reference to different categories. In particular, some examples are described with reference to methods whereas others are described with reference to apparatus. However, a person skilled in the art will gather from the description that, unless otherwise notified, in addition to any combination of features belonging to one category, also any combination between features relating to different category is considered to be disclosed by this application. However, all features can be combined to provide synergetic effects that are more than the simple summation of the features.

While the invention has been illustrated and described in detail in the drawings and foregoing description, such illustration and description are to be considered exemplary and not restrictive. The invention is not limited to the disclosed embodiments. Other variations to the disclosed embodiments can be understood and effected by those skilled in the art, from a study of the drawings, the disclosure, and the appended claims.

The mere fact that certain measures are recited in mutually different dependent claims does not indicate that a combination of these measures cannot be used advantageously.

Any reference signs in the claims should not be construed as limiting the scope.

## Claims

1. A method for monitoring patient state during a medical imaging procedure consisting of an MR, CT, Xray procedure or when on an RT-table, the method comprising:
obtaining pressure signals from at least one optical waveguide pressure sensor array arranged to interact with the patient during an image acquisition process; and
processing the pressure signals to monitor patient state.

2. The method of claim 1, wherein processing the pressure signals comprises detecting patient motion.

3. The method of any preceding claim, wherein obtaining the pressure signals from the at least one optical waveguide pressure sensor array comprises obtaining pressure signals from at least first and second said optical waveguide pressure sensor arrays arranged to interact with the patient.

4. The method of any of claims 2-3, wherein detecting patient motion comprises detecting patient respiratory activity.

5. The method of any of claims 2-4, wherein detecting patient motion comprises detecting patient cardiac activity.

6. The method of any of claims 2-5, wherein detecting patient motion comprises detecting patient cardiorespiratory activity.

7. The method of any of claims 2-6, wherein detecting patient motion comprises detecting patient respiratory activity and patient cardiac activity simultaneously.

8. The method of any preceding claim, wherein processing the pressure signals to monitor patient state comprises determining one or more patient body measurements.

9. The method of claim 8, wherein the body measurements comprise patient body weight.

10. The method of claim 8 or 9, wherein the body measurements comprise one or more geometric measurements.

11. The method of any preceding claim, wherein processing the pressure signals to monitor patient state comprises determining a level of filling of one or more patient organs.

12. The method of any preceding claim, wherein processing the pressure signals to monitor patient state comprises comparing a first patient position determined at a first timepoint with a second patient position determined at a second timepoint to identify a deviation in patient position.

13. The method of any of claims 2-12, wherein detecting patient motion comprises detecting patient motion in two or more dimensions.

14. A patient state monitoring system configured to perform the method of any preceding claim.
